# EUROPEAN PATENT APPLICATION

(11) **EP 1 811 016 A1**
(43) Date of publication of application: **25.07.2007**
(21) Application number: 05800545.5
(22) Date of filing: 02.11.2005
(51) Int. Cl.: C12M 1/26, C12M 1/34, C12Q 1/04, G01N 33/569

(54) **MEMBRANE FILTER FOR MICROBE DETECTION**

(30) Priority: 02.11.2004 JP 2004319479
(71) Applicant: Asahi Breweries, Ltd., Tokyo 104-8323 (JP)
(72) Inventor: Motoyama, Yasuo, R&D Planning Department, Sumida-ku, Tokyo 1308602 (JP); Yasuhara, Takaomi, R&D Planning Department, Sumida-ku, Tokyo 1308602 (JP)
(74) Representative: Eisenführ, Speiser & Partner
(86) International application number: PCT/JP2005/020195
(87) International publication number: WO 2006/049200

(57) **Abstract**

A membrane filter for microbe detection that realizes low fluorescent background, being applicable to staining combining fluorescent dyes of variable wavelengths, and that attains enhanced operationality. There is provided a membrane filter for microbe detection, comprising a polymer layer of 10 to 500 *µ* m thickness and, superimposed thereon, a metal layer of 0.1 nm to 1 *µ* m thickness. Further, there is provided a method of microbe detection wherein microbe detection is carried out with the use of the membrane filter for microbe detection according to any one of claims 1 to 5.

## Description

### Technical Field

The present invention relates to a membrane filter for the detection of microorganisms, which has a low background fluorescence intensity and whose surface is smooth.

### Background Art

There has been known a method which comprises the steps of filtering off microorganisms by the use of a membrane filter, staining the microorganisms trapped on the membrane filter with a fluorescent substance and then observing the microorganisms with a fluorescence microscope. However, this method has a problem that the membrane filter per se also emits fluorescence, and accordingly the method can never detect microorganisms with a high sensitivity. Examples of methods for the observation of such microorganisms include a method in which they are observed with the naked eye and a method comprising taking the image of the microorganisms with a CCD camera. In both of these methods, however, microorganisms cannot be detected with a high sensitivity in a case where it is difficult to discriminate between the background fluorescence and the fluorescence originating from the microorganisms concerned.

To solve the foregoing problems, it is necessary that the membrane filter be stained with a black dye such as Irgalan Black to thus reduce the background fluorescence intensity, or to use a commercially available membrane filter stained with a black dye in advance (such as one available from Whatman under the trade name of Cycloblack^{™}). Although the use of such a previously stained membrane filter would certainly result in the achievement of a background fluorescence-reducing effect with respect to some specific wavelengths, the background fluorescence emitted from the stained membrane filter is not always low enough over the entire fluorescent wavelength region. More specifically, a problem arises such that microorganisms cannot be stained with a combination of fluorescent dyes capable of emitting fluorescence of a variety of wavelengths according to any conventional methods, and accordingly the testing methods which can be used are limited.

On the other hand, the materials used for forming the membrane filter well adapted for the foregoing fluorescent staining are in general polycarbonates and polyesters, but these materials have a variety of problems in that they are soft and quite susceptible to rolling, that the surface of the membrane filter is correspondingly quite easily wrinkled and that the position of the focus is shifted each time the observed field is changed, in a case of microscopic observation. Moreover, a problem further arises such that these materials can be handled only with great difficulty when picking up the membrane filter with a pincette.

### Disclosure of the Invention

### Problems that the Invention is to Solve

Accordingly, it is an object of the present invention to provide a membrane filter for the detection of microorganisms which has a low background fluorescence intensity, which can be adapted the staining with a combination of fluorescent dyes capable of emitting fluorescence of various wavelengths and which is improved in handleability.

### Means for Solving the Problems

The inventors of the present invention have conducted various studies to accomplish the foregoing objects, and as a result have found that the foregoing problems can be efficiently solved by coating the surface of a membrane filter with a metal and have thus completed the present invention.

Accordingly, the present invention herein provides a membrane filter for the detection of microorganisms which comprises a polymer layer having a thickness in the range of from 10 to 500 µm and a metal layer which is formed on the foregoing polymer layer and which has a thickness in the range of from 0.1 nm to 1 µm.

Moreover, the present invention likewise provides a method for the detection of microorganisms which comprises the step of detecting the microorganisms using a membrane filter for the detection of microorganisms as set forth in any one of claims 1 to 5.

### Best Mode for Carrying Out the Invention

The membrane filter for the detection of microorganisms according to the present invention comprises a polymer layer and a metal layer.

The polymer constituting the polymer layer is not restricted to any particular one so long as it is a polymer material currently used for forming a membrane filter and examples thereof include nitrocellulose, polycarbonate, polyester, polysulfone, polyfluoroethylene, polyethylene and polypropylene. Preferably used herein are polyesters and polycarbonates. The membrane filters prepared from polyesters, polycarbonates and the like comprise fine pores having a uniform size, and therefore they are quite suitably used for trapping bacteria and for the observation of the trapped bacteria while staining the same with a fluorescent dye.

The polymer layer may be formed according to any conventionally used method and it is also possible to use any commercially available membrane filter such as Cycloblack^{™} (available from Whatman Corporation) and Nuclepore^{™} (available from Whatman Corporation) as the polymer layer for the membrane filter of the present invention.

The thickness of the polymer layer preferably ranges from 10 to 500 µm and more preferably 20 to 30 µm.

The metal constituting the metal layer is not likewise restricted to any specific one and examples thereof include metals such as gold, silver, copper, zinc, aluminum, titanium, tantalum, chromium, iron, nickel, cobalt, lead and tin; or alloys of these metals (provided that the metal layer is not constituted by Au, Pt, Pd, and Pt-Pd). Preferably used herein is tin. The use of tin is particularly effective, since tin is free of any toxicity to microorganisms and it makes the observation of bacteria after staining the same easy.

The method for forming such a metal layer on the foregoing polymer layer is not limited to any specific one so long as it may allow the formation of a smooth film and specific examples thereof include the evaporation method, the sputtering method and the CVD method.

The thickness of the metal layer is preferably in the range of from 0.1 nm to 1 µm and more preferably 10 to 100nm. If the thickness of the metal layer falls within the range specified above, the metal layer permits the realization of a membrane filter possessing a reduced background fluorescence intensity, a smoother surface and excellent handleability.

The fine pore size, φ, of the membrane filter according to the present invention is preferably in the range of from 0.1 to 10 *µ* m, but the pore size is preferably selected depending on the size of each particular microbial cell used as a subject.

The density of the fine pores for the membrane filter is preferably in the range of from 10⁶ to 10⁸ pores/m². Such a membrane filter shows a tendency that the higher the fine pore density, the better the filtering capacity of the filter, but the lower the strength of the same. Accordingly, one should select a membrane filter having a fine pore size and a fine pore density which takes these characteristic properties into account.

The membrane filter according to the present invention may likewise be prepared by first forming a metal layer on a polymer layer and then forming fine pores on the resulting assembly or it may be prepared by forming a metal layer on a polymer layer on which fine pores have been formed in advance.

The method for forming fine pores may be any one of those currently used and, for instance, includes a method comprising the steps of irradiating a polymer layer with charged particles or neutrons to thus form trajectories thereof through the layer and then chemically etching the trajectories. It would be better to form such fine pores in such a manner that they have a uniform pore size and cylindrical pore channels formed through the film which are arranged in a direction perpendicular to the surface thereof. In this connection, when forming the metal layer according to, for instance, the evaporation technique, the fine pore size of the membrane filter according to the present invention is smaller than that of the polymer layer, and therefore it is necessary to form fine pores through the polymer layer in such a manner that they have a pore size greater than that desired for the ultimate membrane filter.

The detection of microorganisms by using the membrane filter for the detection of microorganisms according to the present invention can be carried out according to the same procedures used in the detection of microorganisms using the conventional membrane filter. More specifically, microorganisms are filtered off through the membrane filter for the detection of microorganisms according to the present invention, the microorganisms trapped on the membrane filter are stained with a fluorescent dye and then they are observed by a fluorescence microscope. The fluorescent staining operation according to the method of the present invention may be carried out using any conventionally used method for staining microorganisms with a fluorescent dye and specific examples thereof include methods each comprising staining microorganisms with, for instance, Escherichia coli-specific fluorescent probe (Vermicon AG) and/or DAPI (4'6diamino-2-phenyl indole).

### Examples

### (Example 1)

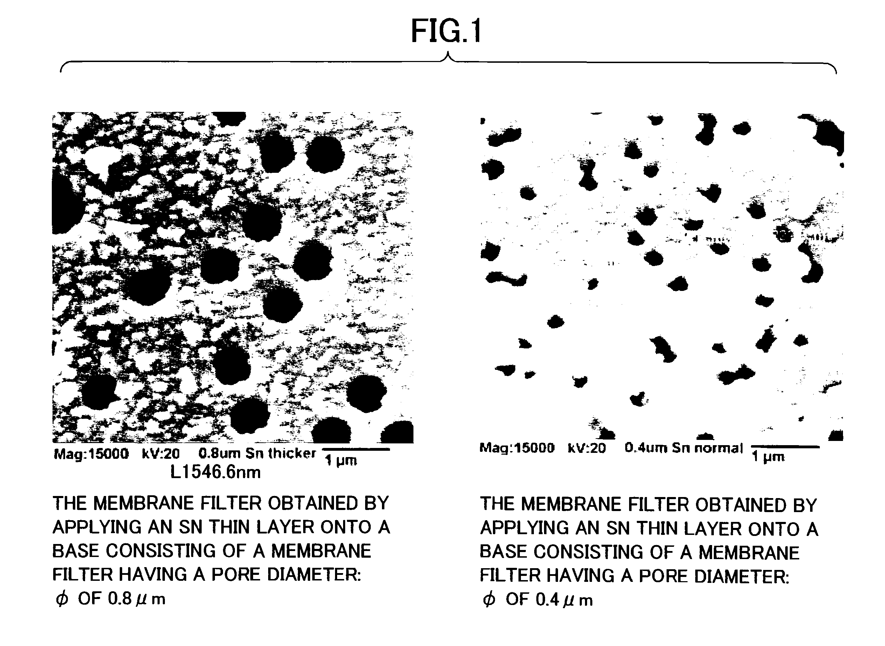

A membrane filter (Cycloblack^{™} available from Whatman Corporation having a diameter of 25 mm and a pore size, φ, of 0.8 *µ* m) was coated with an Sn according to the sputtering technique. This coating step permitted the formation of an Sn layer on the surface of the membrane filter, and the metal-coated membrane filter was found to have an ultimate pore size, φ, of 0.55 µm which was equal to that of the membrane filter currently used in the test of microorganisms and smaller than the foregoing pore size, φ, of 0.8 µm (see, Fig. 1).

Moreover, in a case where a membrane filter (Cycloblack^{™} available from Whatman Corporation) having a pore size, φ, of 0.6 µm was likewise coated with an Sn, the resulting metal-coated membrane filter was found to have an ultimate pore size, φ, of 0.45 µm which was equal to that of the membrane filter currently used in the test of microorganisms.

In a case where a membrane filter (Cycloblack^{™} available from Whatman Corporation) having a pore size, φ, of 0.4 µm was likewise coated with an Sn, the resulting metal-coated membrane filter was found to have an ultimate pore size, φ, of 0.25 µm (see, Fig. 1).

### (Example 2)

The membrane filter obtained in Example 1 by coating a membrane filter (Cycloblack^{™} available from Whatman Corporation having a diameter of 25 mm, and a pore size, φ , of 0.8 µm) with an Sn was inspected for background fluorescence intensity encountered when observing the same with a fluorescence microscope. The membrane filters used in this test were as follows: Cycloblack^{™} available from Whatman Corporation as Commercially Available Membrane Filter 1; Cyclopore^{™} (black) available from Whatman Corporation as Commercially Available Membrane Filter 2; and Cyclopore^{™} (plain) available from Whatman Corporation as Commercially Available Membrane Filter 3. In addition, the membrane filter according to the present invention was one prepared by coating Commercially Available Membrane Filter 1 with an Sn layer by the ion-sputtering technique.

The method of measuring the background fluorescence intensity was as follows: there was fixed, on the observation zone of a fluorescence microscope (DMRXA2 available from Leica Corporation), either one of the coated membrane filters according to the present invention or the membrane filters each free of any coated layer. Respective fluorescent images of these membrane filters were taken through Fluorescent Filter Block A4 (excitation filter: BP360/40; absorption filter: BP470/40); L5 (excitation filter: BP480/40; absorption filter: BP527/30); and Y3 (excitation filter: BP535/50; absorption filter: BP610/75), using a cooled CCD camera (CoolSNAP). The photographing time (exposure time) was set at 60 ms (A4); 160 ms (L5); and 160 ms (Y3). The intensities of the background fluorescence of these respective membrane filters thus photographed were calculated in terms of the averaged luminance per picture element (gradations of 256) for the fluorescent images per image plane (1300 X 1300 picture elements), with the assistance of an image-analysis software: Leica-Qwin. As a result, the highest value was observed for Commercially Available Membrane Filter 3, and accordingly the background fluorescence values were calculated in terms of the values relative to that observed for the Membrane Filter 3, which was defined to be 100.

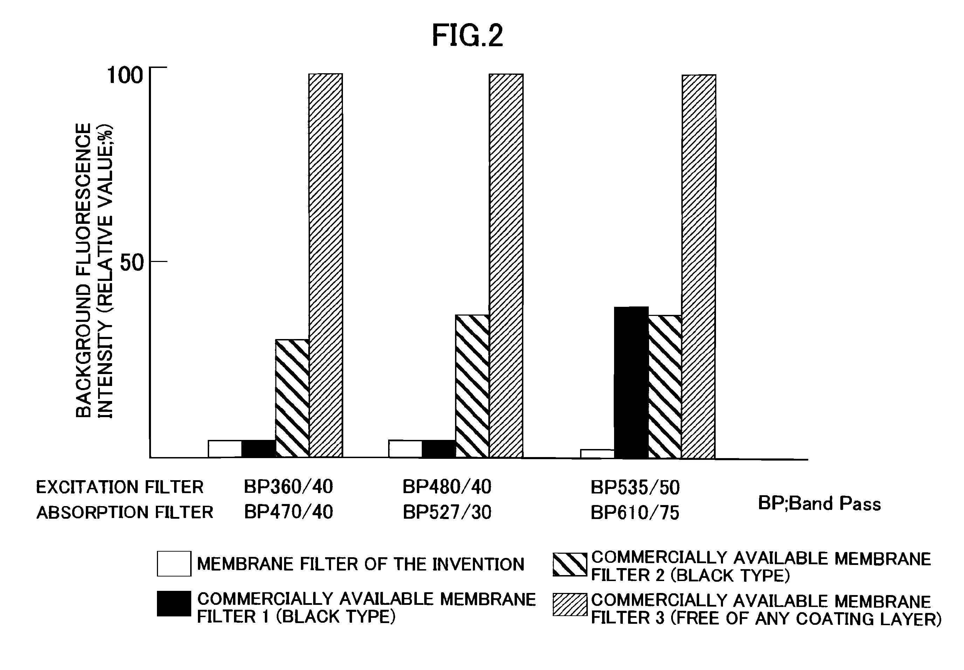

As shown in Fig. 2, these test results clearly indicate that the membrane filter coated with an Sn layer has a significantly low background fluorescence value as compared with those observed for the membrane filters each free of any metal coating layer.

### (Example 3)

Escherichia coli were filtered through the membrane filter prepared in Example 1 obtained by coating a membrane filter (Cycloblack^{™} available from Whatman Corporation having a diameter of 25 mm and a pore size, φ, of 0.8 µm) with an Sn, and then the membrane filter carrying the bacterial cells trapped thereon were cultivated in an STA culture medium at 37 °C for 4 hours. Escherichia coli trapped on the membrane filter and cultivated for such a short period of time were fixed with ethanol and then stained with a commercially available Escherichia coli-specific fluorescent probe (Vermicon AG). The staining was carried out according to the protocol annexed to the probe. Thereafter, Escherichia coli were dyed with DAPI (4'6diamino-2-phenyl indole), followed by the adhesion of the membrane filter to a slide glass, the sealing of the same with a sealing agent: SlowFade^{™} (Molecular Probe), and the subsequent covering of the membrane filter with a cover glass to thus seal the same. This sample was set on the stage of a fluorescence microscope BX60 (available from Olympus Optical Co., Ltd.) and a CCD camera C5810 (available from Hamamatsu Photonics Co., Ltd.) equipped with an objective lens having a magnification of 60 was used to take micrographs.

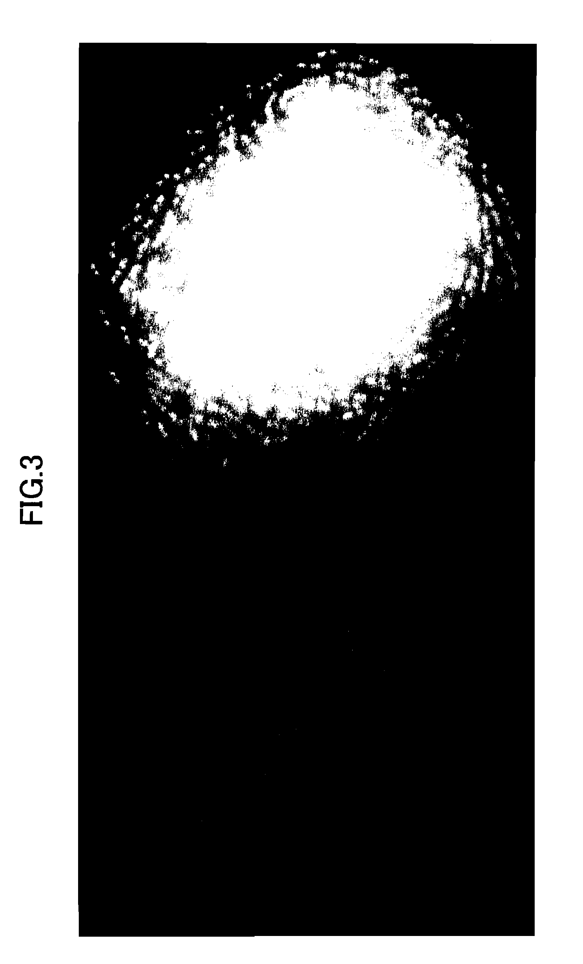

Fig. 3 shows the Escherichia coli stained with an Escherichia coli-specific probe (on the left-hand side) and the Escherichia coli stained with a DAPI (on the right-hand side), both of them being found in the same field.

As will be seen from this figure, Escherichia coli were distinctively stained by either of these staining methods and these results clearly indicate that the staining methods are effective.

### Brief Description of the Drawings

[Figure 1] This figure shows observed surface images of a membrane filter according to the present invention obtained using an electron microscope.
[Figure 2] This figure shows background fluorescence observed for commercially available membrane filters and the membrane filter according to the present invention.
[Figure 3] This figure shows a micrograph showing an Escherichia coli trapped on the membrane filter according to the present invention and subjected to fluorescent staining.

## Claims

1. A membrane filter for the detection of microorganisms comprising a polymer layer having a thickness in the range of from 10 to 500 *µ* m and a metal layer which is formed on the said polymer layer and which has a thickness in the range of from 0.1 nm to 1 µm (provided that the metal layer is not constituted by Au, Pt, Pd, and Pt-Pd).

2. The membrane filter for the detection of microorganisms of claim 1, wherein the fine pore size, φ, of the membrane filter in the range of from 0.1 to 10 µm.

3. The membrane filter for the detection of microorganisms of claim 1 or 2, wherein the density of the fine pores for the membrane filter in the range of from 10⁵ to 10⁸ pores/m².

4. The membrane filter for the detection of microorganisms of any one of claims 1 to 3, wherein the metal layer contains Sn.

5. A method for the detection of microorganisms comprising the step of detecting the microorganisms using a membrane filter for the detection of microorganisms of any one of claims 1 to 4.
